# EUROPEAN PATENT SPECIFICATION

(11) **EP 3 448 231 B1**
(45) Date of publication and mention of the grant of the patent: **02.07.2025**
(21) Application number: 17724322.7
(22) Date of filing: 27.04.2017
(51) Int. Cl.: A61B 3/14, A61B 3/00

(54) **METHOD OF CREATING AND PROCESSING A COLOR IMAGE OF AN IRIDOCORNEAL REGION OF AN EYE AND APPARATUS THEREFOR**
VERFAHREN ZUR ERZEUGUNG UND VERARBEITUNG EINES FARBBILDES EINER IRIDOKORNEALEN REGION EINES AUGES UND VORRICHTUNG DAFÜR
PROCÉDÉ DE CRÉATION ET DE TRAITEMENT D'UNE IMAGE COLORÉE D'UNE RÉGION IRIDOCORNÉENNE D'UN OEIL ET APPAREIL ASSOCIÉ

(30) Priority: 29.04.2016 IT UA20163001
(43) Date of publication of application: 06.03.2019
(73) Proprietor: Nidek Technologies S.R.L., 20121 Milano (IT); Nidek Co., Ltd., Gamagori, Aichi (JP)
(72) Inventor: DE GIUSTI, Andrea, 35020 Albignasego (PD) (IT); PAJARO, Simone, 35020 Albignasego (PD) (IT)
(74) Representative: De Gregori, Antonella
(86) International application number: PCT/EP2017/060093
(87) International publication number: WO 2017/186863

(56) References cited:
- WO-A1-01/67945
- JP-A- H03 222 937
- US-A1- 2013 226 008

## Description

### TECHNICAL FIELD

The present invention relates to a method of creating and processing a color image of an iridocorneal region of an eye.

### BACKGROUND ART

Ophthalmologists observe eyes and their parts in order to check their health status, to diagnose any diseases and, if necessary, to choose treatments.

Ophthalmologists perceive color images of the eyes and their parts, and often base their diagnoses on the colors of the eye parts.

Anyway, the perception of colors by ophthalmologists is influenced by objective and subjective factors. The quality of a diagnosis on an eye strongly depends on the apparatus that was used for observing the eye and its parts as well as on the skill of the ophthalmologist who made the diagnosis.

From document US 2013/226008 A1 are known methods, systems and computer readable storage devices for determining a color score for at least a portion of a biological tissue by obtaining a digital image of the biological tissue, receiving a selection of a portion of the image as an evaluation area, determining for each of a plurality of pixels within the evaluation area, a plurality of color components that are based on a Cartesian color space, determining, from the color components, a hue value in a polar coordinate based color space, determining a color value based on the hue value for each of the plurality of pixels, and assigning a color score to the evaluation area based on an average of the color values of the plurality of pixels.

However, as already mentioned above, also the solution according to US 2013/226008 A1 is device-dependent (there is one color in the image that is correct, i.e. the color used for balancing, but no guaranties are provided regarding the other colors of the image, i.e. the color dynamic of the image).

Therefore, it would be desirable to provide ophthalmologists with methods and apparatuses that assist them in the objective evaluation of the colors of eye parts, in particular of the iridocorneal regions of eyes as it is difficult to observe such regions well.

### SUMMARY

The basic idea behind the present invention provides for selecting at least one sub-image within a detected image corresponding to a region of interest, computing at least one statistic parameter of the selected sub-image, and determining an indicator based on the computed statistic parameter.

One or more of such indicators may be provided to the ophthalmologist as examination result or results.

It is important that the detection of color images is preceded by calibrating a pair of at least one color image detector device and at least one color illuminator device. Calibration may be carried out only once (for example when the medical apparatus used for image detection is manufactured) or repeatedly (for example once a year).

The color images are detected in a device-dependent color space (being a RGB color space) and then transformed in a device-independent and perceptually-uniform color space (being the CIE LAB color space).

A first important idea behind the present invention provides for computing a computed color (for example an average color) of the sub-image, computing a distance between the computed color and a comparison color (for example a "reference red" color), and providing the distance as an examination result.

A second important idea behind the present invention provides for detecting a plurality (at least two) of color images of an iridocorneal region of an eye at different times (for example every 5-5000 ms) and comparing them. Before, such detections, fluorescein is administered to the person under examination.

A third important idea behind the present invention is to use a predetermined grading scale (for example Scheie's angle pigmentation scale) for determining the indicator.

The present invention is defined by the appended claims that have to be considered an integral part of the present description.

A first aspect of the present invention corresponds to a method of creating and processing a color image of an iridocorneal region of an eye.

A second aspect of the present invention, which is not part of the claimed subject-matter, corresponds to an apparatus for creating and processing a color image of an iridocorneal region of an eye.

Processing of color images of iridocorneal regions of eyes may be carried out for example in the medical apparatus that detects the images, in a computer connected to the medical apparatus that detects the images, in a computer non-connected to the medical apparatus that detects the images. In the last case, images may be stored for example in a memory device to be read by or connected to the computer.

A third aspect of the present invention, which is not part of the claimed subject-matter, corresponds to a computer program product for processing color images of iridocorneal regions of eyes. Such computer program products may be downloaded from a communication network and/or stored on a computer-readable medium and/or stored in a processor-readable medium of a processing unit.

### BRIEF DESCRIPTION OF DRAWINGS

The accompanying drawings, which are incorporated herein and constitute a part of the specification, illustrate exemplary embodiments of the present invention and, together with the detailed description, explain these embodiments. In the drawings:
Fig.1 shows a simplified cross-section view (Fig.1A) and a simplified front view (Fig.1B) of an eye,
Fig.2 shows a simplified block diagram of an embodiment of an apparatus according to the present invention,
Fig.3 shows a much-simplified block diagram of a first embodiment of a processing system according to the present invention to be used with a first embodiment of an apparatus according to the present invention,
Fig.4 shows a much simplified block diagram of a second embodiment of a processing system according to the present invention to be used with a second embodiment of an apparatus according to the present invention, and
Fig.5 shows a much-simplified block diagram of a third embodiment of a processing system according to the present invention to be used with a third embodiment of an apparatus according to the present invention.

### DETAILED DESCRIPTION

The following detailed description of exemplary embodiments refers to the accompanying drawings.

The following detailed description does not limit the present invention. Instead, the scope of the present invention is defined by the appended claims.

Reference throughout the specification to "one embodiment" or "an embodiment" means that a particular feature, structure, or characteristic described in connection with an embodiment is included in at least one embodiment of the subject matter disclosed. Thus, the appearance of the phrases "in one embodiment" or "in an embodiment" in various places throughout the specification is not necessarily referring to the same embodiment. Further, the particular features, structures or characteristics may be combined in any suitable manner in one or more embodiments.

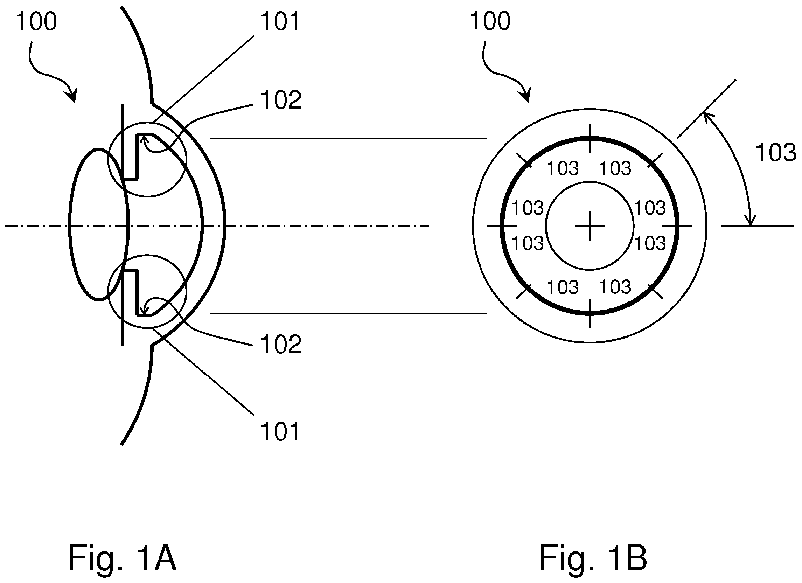

In Fig.1, an eye is labeled 100 and its iridocorneal annular zone 101; considering Fig.1A, as known, the iridocorneal annular zone 101 is substantially delimited on the left side by the iris, on the right side by an annular portion of the cornea, and on the intermediate side by the trabecular meshwork and adjacent tissues 102.

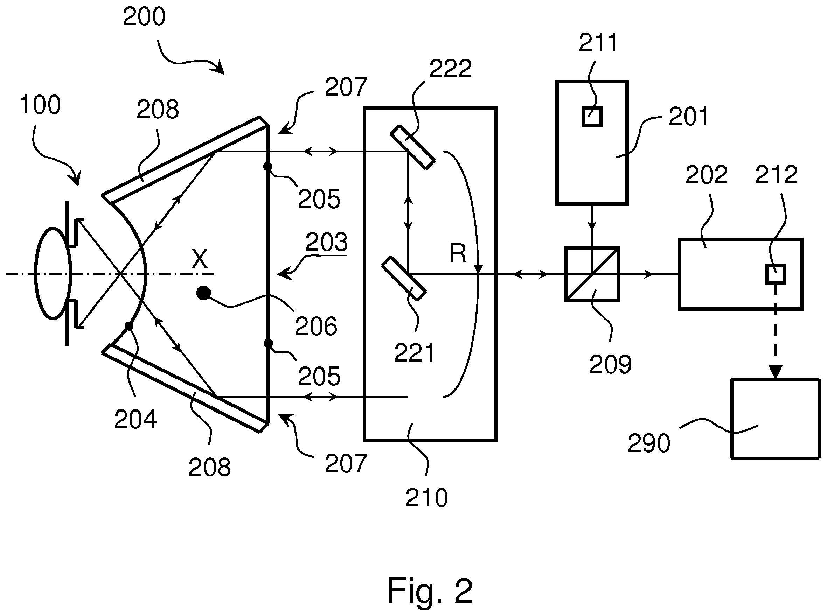

An apparatus according to the present invention, such as the one shown in Fig. 2, is designed for observing of at least a portion of an iridocorneal annular zone of an eye, detecting one or more color images of the region, processing the images and providing one or more examination results. Preferred embodiments of such apparatus allow observation of the whole annular zone, i.e. the annular portion has an amplitude of 360° as in Fig.1B. Alternative embodiments of such apparatus may allow observation for example of one portion having amplitude of e.g. 180° or 120° or 90°, or of a set of separate portions having amplitude of e.g. 45° or 30° or 15°.

The status of the tissues in the iridocorneal zone of the eye is relevant but not limited to the diagnosis of for example primary open-angle glaucoma, angle-closure glaucoma, normal tension glaucoma, low tension glaucoma, secondary glaucoma, primary congenital glaucoma, inflammatory glaucoma, lens-related glaucoma, traumatic glaucoma, iridocorneal endothelial (ICE) syndrome, glaucoma in phacomatoses, iridocorneal dysgenesis, ghost cell glaucoma, glaucoma in cavernous sinus fistula, glaucoma in intraocular tumors, glaucoma in ciliochoroidal detachment, Glaucoma in epithelial ingrowth, glaucoma in iridoschisis, and, in general, ocular hypertension, trabecular meshwork damages, neovascularization of the iridocorneal structures, anomalies of the ciliary body, iris damages and pathogenic strains.

The 360° portion of the iridocorneal zone of Fig.1B is divided in a number of adjacent sub-portions 103; in particular, by way of example, it is divided in eight sub-portions 103 having the same amplitude of 45°. It is to be noted that according to alternative embodiments, the number of sub-portions may be different, for example from e.g. four to e.g. thirty-six.

The observation and detection portion of the apparatus of Fig. 2 is described in detail in international patent application published as WO2015180923A1 (see its Fig. 6).

Alternative observation and detection arrangements that may be used for implementing the present invention are shown and described in detail in international patent application published as WO2015180923A1.

It is to be noted that the present invention may be implemented through observation and detection arrangements different from those shown and described in WO2015180923A1.

Natural light that is rather diffused (i.e. coming from different directions) and rather weak does not allow a good observation of the iridocorneal annular zone of an eye. Therefore, according to the present invention, artificial light is much preferably used.

The apparatus of Fig.2 (largely corresponding to Fig. 6 of document WO2015180923A1) is suitable for observation of the whole iridocorneal annular zone of an eye and comprises: a stationary illumination assembly consisting of one illumination electric (in particular electronic) device 201, a stationary image capturing assembly consisting of one image capturing electric (in particular electronic) device 202, and a stationary front optical assembly 203; the illumination device 201 has a light emitter 211; the image capturing device 202 has a light sensor 212 consisting of a matrix of light detectors.

According to alternative embodiments of the present invention, the apparatus may comprise a plurality of illumination devices and/or a distinct plurality of image capturing devices (see e.g. Fig. 2 of document WO2015180923A1). The light sensors of the image capturing devices may be separate sensors or portions of a single large light sensor.

The image capturing assembly of Fig. 2 is used for detecting color images of iridocorneal regions of eyes. The image capturing device 202 of the assembly comprises of a bi-dimensional array of pixels. The color images are in a color space defined by three color dimensions thus a color of each pixel of the array is associated to three color components,

In the embodiment of Fig. 2, the illumination device 201 is successively associated to a distinct and different illumination optical path that goes to a corresponding sub-portion (see e.g. elements 103 in Fig.1) of the iridocorneal annular zone of an eye.

In the embodiment of Fig. 2, the image capturing device 202 is successively associated to a distinct and different imaging optical path that comes from a corresponding sub-portion (see e.g. elements 103 in Fig.1) of the iridocorneal annular zone of an eye.

In the embodiment of Fig. 2, the front optical assembly 203 has a front surface 204 designed to be located close, i.e. at a short distance, to the front surface of an eye 100, a rear surface 205 designed to be located far, i.e. at a long distance, from the front surface of an eye 100; a viscous optical coupling agent, for example an ophthalmic gel, is applied on the front surface of the optical assembly and/or to the outside surface of the cornea before applying the optical assembly to the eye. According to a good practice, the above-mentioned "short distance" between the front surface of the assembly and the front surface of the eye should be the range 0.5-2.5 mm, preferably in the range 1.0-2.0 mm; shorter distances and longer distances have preferably to be avoided; in particular contact between the assembly and the eye has preferably to be avoided. The above-mentioned "long distance" depends on the length of front optical assembly and is preferably in the range 1-4 cm, more preferably in the range 1.5-2.5 cm.

In the embodiment of Fig. 2, the assembly 203 comprises a central portion 206 located between the front surface 204 and the rear surface 205 and a lateral portion 207 located around the central portion 206 and surrounding it completely (i.e. it is 360° wide). According to this embodiment, the central portion 206 is a solid transparent prism having the shape of truncated octagonal pyramid; the front surface 204 is concave (corresponding to the convex outside surface of the cornea); according to this embodiment, the lateral portion 207 consists of a single-piece reflecting element 208 adjacent to the lateral surface of prism so that it has eight reflecting surfaces facing the central portion 206.

The equipment of Fig.2 comprises also a rear optical assembly 210 and one stationary beam splitter 209.

The rear optical assembly 210 has two functions distinct from each other: rotating the illumination light beam coming from the illumination device 201 and going to the eye 100 and rotating the imaging light beam coming from the eye 100 and going to the image capturing device 202. Both these rotations are around the symmetry axis X of the front optical assembly 203.

The rear optical assembly is rotary in the sense that some of its components are arranged to rotate, in particular to carry out a rotation motion (R in Fig.2) around the symmetry axis X of the front optical assembly. According to this embodiment there is a central mirror 221 and a lateral mirror 222; both mirrors 221 and 222 rotate around the symmetry axis X of the front optical assembly 203 step-by-step by e.g. 45° and correspondingly e.g. eight images are successively created on the sensor 212; properly, the illumination light beam and the imaging light beam are rotated around the symmetry axis X of the front optical assembly 203 in their way between the eye 100 and the central mirror 221 and, parallelly, between the reflecting element 208 and the lateral mirror 222.

According to the embodiment of Fig. 2, the assembly 203 and all its components (in particular, the central portion 206 and the lateral portion 207) is stationary; this means that a panoramic image of a whole iridocorneal annular zone of an eye may be obtained without moving it.

The color images detected through the image capturing assembly of apparatus 200 are then processed.

In the embodiment of Fig. 2, the images detected by the light sensor 212 are conceptually (see dashed line) transferred to an image processing system 290; there are various transferring possibilities. It is to be noted that a preliminary (analog and/or digital) processing may be carried out inside the image capturing device 202.

According to alternative embodiments of the present invention, images detected by a plurality of light sensors are conceptually transferred to an image processing system either contemporaneously (for example parallel connection) or sequentially (for example bus connection).

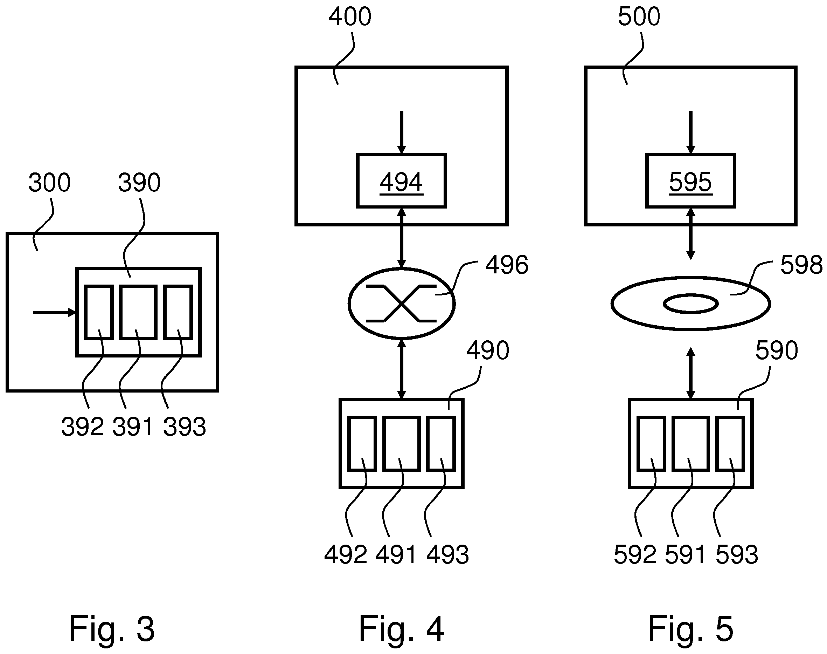

According to the embodiment of Fig. 3, the image processing system 390 is inside the apparatus 300. System 390 comprises for example a processing unit 391 (for example a microprocessor connected to some memory), an input device 392 (for example a keyboard and/or a mouse) and output device 393 (for example a screen and/or a printer and/or a CD/DVD burner), connected together. There is a connection between image capturing device 202 and image processing system 390 that is internal to apparatus 300.

According to the embodiment of Fig. 4, the image processing system 490 is outside the apparatus 400. System 490 may be for example a PC and comprises for example a processing unit 491 (for example a microprocessor connected to some memory), an input device 492 (for example a keyboard and/or a mouse and/or a reception device) and output device 493 (for example a screen and/or a printer and/or a CD/DVD burner), connected together. There is a connection between image capturing device 202 and image processing system 490. Such connection may be a simple connection embodied by e.g. an electric cable or a complex connection embodied by e.g. an Internet connection (see element 496 in Fig. 4). In order to transfer color images externally, apparatus 400 comprises a transmission device (see element 494 in Fig. 4); the transmission device is internally directly connected to image capturing device 202. On the other side, system 490 comprises a reception device (see element 492 in Fig. 4).

According to the embodiment of Fig. 5, the image processing system 590 is outside the apparatus 500. System 590 may be for example a PC and comprises for example a processing unit 591 (for example a microprocessor connected to some memory), an input device 592 (for example a keyboard and/or a mouse and/or a CD/DVD reader) and output device 593 (for example a screen and/or a printer and/or a CD/DVD burner), connected together. According to this embodiment, images are transferred through a memory device, for example a CD/DVD (see element 598 in Fig. 5). In order to transfer color images externally, apparatus 500 comprises a CD/DVD burner (see element 595 in Fig. 5); the CD/DVD burner is internally directly connected to image capturing device 202. On the other side, system 590 comprises a CD/DVD reader (see element 592 in Fig. 5).

In general, the method according to the present invention comprises the preliminary step of:
- calibrating a pair of at least one color image detector device (see e.g. device 202 in Fig. 2) and at least one color illuminator device (see e.g. device 201 in Fig. 2);
and the examination steps of:
A) detecting at least one color image of an iridocorneal region (see e.g. element 101 in Fig. 1) of an eye (see e.g. element 100 in Fig. 1) through the detector device (see e.g. device 202 in Fig. 2) consisting of a bi-dimensional array of pixels, wherein the at least one color image is in a color space defined by three color dimensions thus a color of each pixel of said bi-dimensional array is associated to three color components,
B) selecting at least one sub-image within said detected image corresponding to a region of interest,
C) computing at least one statistic parameter of said selected sub-image, wherein the statistic parameter is selected from the group comprising an average value of a color component, a variance value of a color component, a minimum value of a color component and a maximum value of a color component, a skewness value of a color component, a kurtosis index value of a color component, a distribution of a color component, a distribution gradient of a color component,
D) determining an indicator based on said at least one statistic parameter,
E) providing said indicator as an examination result.

Preferably and typically, the whole imaging chain (i.e. illumination, acquisition and display of images) is calibrated in four successive steps:
1. The display (if present and of interest) is calibrated by using a proper hardware (e.g. a professional display calibration tool) with its proper software; this tool, connected through an USB cable, produces a calibration profile; this calibration profile is stored and then used as the display color settings.
2. The light emitter (or emitters) (typically a LED) is set on a pre-determined power value that provides optimal luminous intensity for the image acquisition.
3. A white balance procedure sets the light emitter (or emitters) to illuminate the light sensor (or sensors) (typically a CCD): a software (typically provided with the detector device) allows to adjust the color channels gain in order to obtain neutral color histograms on the light sensor.
4. A color image of a color checker (e.g. a professional white balance tool) is acquired and verified for a correspondence of the true white color.

Calibration may be carried out only once (for example when the medical apparatus used for image detection is manufactured) or repeatedly (for example once a year).

There are many color spaces. The most widespread are the RGB color space and the CMY color space. Other color spaces were defined for example by CIE [Commission Internationale de l'Eclairage]: the CIE XYZ color space, the CIE LAB color space and the CIE LUV color space.

It is simple and effective to detect images in a device-dependent color space, such as RGB, through an ordinary image detector device (available on the market), and to select sub-images still in a device-dependent color space.

Then it is preferable to transform the sub-image in the device-dependent color space to a sub-image in a device-independent and preferably perceptually-uniform color space, in particular a CIE LAB color space.

In some cases, the region of interest, or "ROI", may correspond to an entire image. In other cases, a single image may comprise more than one region of interest.

The selection of the region of interest may be completely manual or completely automatic or manual but guided by an automatic procedure.

It is to be noted that the same region of interest may provide a first set of (one or more) indicators and a second set of (one or more) examination results. The number of examination results may be different from the number of indicators; in fact, one examination result may be based on some indicators.

It is to be noted that an examination result may be a number and/or a "class"/"grade".

It is to be noted that an examination result may also be an image, for example a (black- and-white or color) processed image, or a graph, for example a linear or circular graph showing a plurality of indicators.

It is to be noted that the method according to the present invention may provide a plurality of examination results.

Many method embodiments lie within the broad definition of the method according to the present invention as set out above.

According to a first category of methods:
step C corresponds to computing at least one computed color of the sub-image,
step D corresponds to computing at least one distance between said at least one computed color and at least one comparison color, and
step E corresponds to providing said at least one distance as an examination result.

A comparison color may be predetermined and, for example, may derive from an examination trial on a plurality of persons.

According to a specific embodiment in this first category, the comparison color is a "reference red" color. The distance may be considered an indicator of the quantity of blood in the tissue.

If for example image processing is carried out in the RGB color space and if for example the comparison color is a "reference red" color, the computation of the average color and of the distance between the comparison color and the average color may take into account even only one color component (i.e. "red") of all the pixels of the sub-image.

If for example image processing is carried out in the CIE LAB or CIE LUV color space and if for example the comparison color is a "reference red" color, computation of the average color and of the distance between the comparison color and the average color takes into account two color components (i.e. A and B or U and V) or three color components of all the pixels of the sub-image.

According to a sub-category of the first category of methods, a plurality (at least two) of color images of an iridocorneal region of an eye at different times is detected, a plurality (at least two) of sub-images are selected (corresponding to the same or substantially the same sub-region), and a plurality (at least two) of average colors are computed and compared between each other.

The time period between successive detections may be for example in the range from 5 ms to 5 s.

Such method may be used for checking the flow of aqueous humor through the trabecular meshwork. In this case, fluorescein is administered to the person under examination before such detections. In the case of gonioscopy, fluorescein appears to be green. Therefore, the level of green of the trabecular meshwork corresponds to the quantity of aqueous humor flowing in the trabecular meshwork. Furthermore, the variation of the level of green of the trabecular meshwork may be considered an indicator of the flow of aqueous humor through the trabecular meshwork.

If for example image processing is carried out in the RGB color space and if for example the comparison color is a "reference green" color, computation of the average color and of the difference or distance between the comparison color and the average color may take into account even only one color component (i.e. "green") of all the pixels of the sub-image.

If for example image processing is carried out in the CIE LAB or CIE LUV color space and if for example the comparison color is a "reference green" color, computation of the average color and of the difference or distance between the comparison color and the average color takes into account two color components (i.e. A and B or U and V) or three color components of all the pixels of the sub-image.

In general, according to this sub-category of the first category, the method comprises at least the examination steps of:
- detecting a second color image of an iridocorneal region of an eye, selecting a second sub-image within said second color image corresponding to a region of interest, and computing a second computed color (for example an average color) of the second sub-image,
- detecting a third color image of an iridocorneal region of the eye, selecting a third sub-image within said third color image corresponding to said region of interest, and computing a third computed color (for example an average color) of the third sub-image;
- computing a ratio between a distance between said third computed color and said second computed color, and a duration of the time period occurring between detecting the second color image and detecting the third color image
- providing said ratio as an examination result.

The method may also comprise the examination step of:
- detecting a first color image of an iridocorneal region of the eye, selecting a first sub-image within said first color image corresponding to said region of interest, and computing a first computed color (for example an average color) of the first sub-image, carried out before detecting the second color image and detecting the third color image; another time period occurs between detecting the first color image and detecting the second color image.

Fluorescein may be administered to the person under examination exactly at the time of detecting the first color image or shortly before.

The first computed color (for example an average color) may be used as a reference color for the person under examination.

A plurality of color image detections and color distance computations may be carried out.

According to a second category of methods, in step D, the indicator is determined based on a predetermined grading scale. In this case, the examination result is a "grade" or "class" in the predetermined grading scale.

The grading scale may be a "qualitative" grading scale while the statistic parameter/parameters used for determining the indicator is/are "quantitative" parameters.

The grading scale may be a known grading scale such as for example Scheie's angle pigmentation scale. This scale is used for visual comparison by ophthalmologists.

The grading scale may be a new grading scale derived from a known grading scale such as for example Scheie's angle pigmentation scale. For example, it turned to be advantageous to use a grading scale wherein: new grade I corresponds to the combination of Scheie's grades 0 and I, new grade II corresponds to Scheie's grades II, new grade III corresponds to the combination of Scheie's grades III and IV.

Classification according to the Scheie's angle pigmentation scale or a modified Scheie's angle pigmentation scale may advantageously be carried out through a statistical classifier (e.g. decision tree, random forest, neural network, support vector machine, etc.); for example, the statistical classifier may be based on a set of statistic parameters, in particular an average value and a kurtosis index value.

More in general, classification may be carried out through a deterministic or statistical classifier based on a set of statistics and/or deterministic (i.e. measured) parameters.

According to a first set of embodiments in the second category of methods,
in step C a plurality of statistic parameters of the sub-image are computed,
in step D the indicator is determined based on the plurality of statistic parameters,
and step D is carried out through a decision tree classifier.

According to an embodiment of the first set, the CIE LAB space is used; the average value of the A component is tested, if avg(A)<=1.825 then the examination result is new grade I else the kurtosis index value of the L component is checked, if kurt(L)<=3.162 then the examination result is new grade II else the examination result is new grade III.

According to a second set of embodiments in the second category of methods,
in step C a plurality of statistic parameters of the sub-image are computed,
in step D the indicator is determined based on the plurality of statistic parameters,
and step D is carried out through a neural network classifier.

In this case, the method may comprise the further a preliminary step of training the neural network classifier only once or repeatedly or continuously.

Especially according to the second category of methods, it may be advantageous that:
in step A a plurality of color images of an iridocorneal region of a same eye at different angles (for example at 0°, 90°, 180° and 270°) are detected,
in step B a corresponding plurality of sub-images are selected within said plurality of color images,
in step C at least one corresponding plurality of statistic parameters of the plurality of sub-images are computed,
in step D a corresponding plurality of indicators are determined based on the at least one plurality of statistic parameters and one predetermined grading scale.

In this case, in step E the plurality of indicators and/or a linear or circular graph showing the plurality of indicators may be provided as examination result. In addition to the indicators and/or graph, a plurality of images may be provided.

An apparatus according to the present invention may comprise means specifically adapted to carry out the method as set out above.

An apparatus according to the present invention may comprise means specifically adapted to carry out the examination at least the steps B, C, D of the method as set out above. Such means correspond for example to systems 290, 390, 490 and 590 respectively in Fig. 2, Fig. 3, Fig. 4 and Fig. 5, in particular units 391, 491 and 591.

Some of the means for carrying out the method are typically software means; indeed, one aspect of the present invention is a "computer program product".

Such product may be downloadable from a communication network, such as for example the Internet.

Such product may be stored on a computer-readable medium, such as for example a CD or DVD.

Such product may be stored in a processor-readable medium of a processing unit, such as for example the memories of units 391, 491 and 591.

## Claims

1. Method of creating and processing a color image of an iridocorneal region (101) of an eye (100) comprising the examination steps of:
A) detecting at least one color image of an iridocorneal region (101) of an eye (100) through a detector device (202) consisting of a bi-dimensional array of pixels, wherein the at least one color image is in a color space defined by three color dimensions thus a color of each pixel of said bi-dimensional array is associated to three color components, wherein the detector device (202) and a color illuminator device (201) are preliminary calibrated as a pair,
B) selecting at least one sub-image within said detected image corresponding to a region of interest,
C) computing at least one statistic parameter of said selected sub-image, wherein the statistic parameter is selected from the group comprising an average value of a color component, a variance value of a color component, a minimum value of a color component and a maximum value of a color component, a skewness value of a color component, a kurtosis index value of a color component, a distribution of a color component, a distribution gradient of a color component,
D) determining an indicator based on said at least one statistic parameter,
E) providing said indicator as an examination result;
wherein step A provides a color image in a device-dependent color space being RGB, and
wherein before step C, there is a step F of transforming the sub-image in the device-dependent color space to a sub-image in a device-independent and perceptually-uniform color space being CIE LAB,
wherein at least steps B, C, D, E and F are carried out by a computer program.

2. Method according to claim 1,
wherein step C corresponds to computing at least one computed color of the sub-image,
wherein step D corresponds to computing at least one distance between said at least one computed color and at least one comparison color,
wherein step E corresponds to providing said at least one distance as an examination result.

3. Method according to claim 2, wherein said at least one comparison color is predetermined.

4. Method according to claim 3, wherein said at least one comparison color derives from an examination campaign on a plurality of persons.

5. Method according to any of the preceding claims from 2 to 4, comprising the examination steps of:
- detecting a second color image of an iridocorneal region (101) of an eye (100), selecting a second sub-image within said second color image corresponding to a region of interest, and computing a second computed color of the second sub-image,
- detecting a third color image of an iridocorneal region (101) of the eye (100), selecting a third sub-image within said third color image corresponding to said region of interest, and computing a third computed color of the third sub-image;
wherein a time period occurs between detecting the second color image and detecting the third color image;
comprising the further examination steps of:
- computing a ratio between a distance between said third computed color and said second computed color, and a duration of said time period
- providing said ratio as an examination result.

6. Method according to claim 5, comprising, before detecting the second color image and detecting the third color image:
- detecting a first color image of an iridocorneal region of the eye, selecting a first sub-image within said first color image corresponding to said region of interest, and computing a first computed color of the first sub-image.
Wherein another time period occurs between detecting the first color image and detecting the second color image.

7. Method according to claim 1,
wherein in step D the indicator is determined based on a predetermined grading scale.

8. Method according to claim 7,
wherein in step C a plurality of statistic parameters of the sub-image are computed,
wherein in step D the indicator is determined based on the plurality of statistic parameters,
wherein step D is carried out through a classifier.

9. Method according to claim 7,
wherein in step C a plurality of statistic parameters of the sub-image are computed,
wherein in step D the indicator is determined based on the plurality of statistic parameters,
wherein step D is carried out through a neural network classifier.

10. Method according to claim 9, comprising further a preliminary step of training the neural network classifier only once or repeatedly or continuously.

11. Method according to claim 7 or 8 or 9 or 10,
wherein in step A a plurality of color images of an iridocorneal region (101) of a same eye (100) at different angles are detected,
wherein in step B a corresponding plurality of sub-images are selected within said plurality of color images,
wherein in step C at least one corresponding plurality of statistic parameters of the plurality of sub-images are computed,
wherein in step D a corresponding plurality of indicators are determined based on the at least one plurality of statistic parameters.

12. Method according to claim 11,
wherein in step E the plurality of indicators and/or a linear or circular graph showing the plurality of indicators are provided as examination result.

## Patentansprüche

1. Verfahren zur Erstellung und Verarbeitung ein Farbbildes eines iridokornealen Bereichs (101) eines Auges (100), umfassend die Prüfschritte:
A) Erfassen mindestens eines Farbbildes eines iridokornealen Bereichs (101) eines Auges (100) durch eine Detektoreinrichtung (202), bestehend aus einem zweidimensionalen Array aus Pixeln, wobei das mindestens eine Farbbild sich in einem Farbraum befindet, der durch drei Farbdimensionen definiert ist, so dass eine Farbe eines jeden Pixels des zweidimensionalen Arrays mit drei Farbkomponenten verknüpft ist, wobei die Detektoreinrichtung (202) und eine Farbbeleuchtereinrichtung (201) als ein Paar vorkalibriert sind,
B) Auswählen von mindestens einem Teilbild innerhalb des erfassten Bildes, das einem Bereich von Interesse entspricht,
C) Berechnen von mindestens einem statistischen Parameter des ausgewählten Teilbildes, wobei der statistische Parameter ausgewählt ist aus der Gruppe bestehend aus einem Durchschnittswert einer Farbkomponente, einem Varianzwert einer Farbkomponente, einem Mindestwert einer Farbkomponente und einem Höchstwert einer Farbkomponente, einem Schiefewert einer Farbkomponente, einem Kurtosisindexwert einer Farbkomponente, einer Verteilung einer Farbkomponente, einem Verteilungsgradient einer Farbkomponente,
D) Bestimmen eines Indikators basierend auf mindestens einem statistischen Parameter,
E) Bereitstellen des Indikators als ein Prüfergebnis;
wobei Schritt A ein Farbbild in einem geräteabhängigen RGB-Farbraum bereitstellt, und
wobei vor dem Schritt C ein Schritt F des Umwandelns des Teilbildes in dem geräteabhängigen Farbraum in ein Teilbild in einem geräteunabhängigen und wahrnehmungseinheitlichen Farbraum, der CIE LAB ist, erfolgt,
wobei mindestens die Schritte B, C, D, E und F durch ein Computerprogramm ausgeführt werden.

2. Verfahren nach Anspruch 1,
wobei Schritt C dem Berechnen von mindestens einer berechneten Farbe des Teilbildes entspricht, wobei Schritt D den Berechnen von mindestens einem Abstand zwischen der mindestens einen berechneten Farbe und mindestens einer Vergleichsfarbe entspricht,
wobei Schritt E dem Bereitstellen des mindestens einen Abstands als ein Prüfergebnis entspricht.

3. Verfahren nach Anspruch 2, wobei die mindestens eine Vergleichsfarbe vorgegeben ist.

4. Verfahren nach Anspruch 3, wobei die mindestens eine Vergleichsfarbe aus einer Prüfkampagne an einer Vielzahl von Personen stammt.

5. Verfahren gemäß einem der vorstehenden Ansprüche 2 bis 4, umfassend die Prüfschritte:
- Erfassen eines zweiten Farbbildes eines iridokornealen Bereichs (101) eines Auges (100), Auswählen eines zweiten Teilbildes innerhalb des zweiten Farbbildes, das einem Bereich von Interesse entspricht, und Berechnen einer zweiten berechneten Farbe des zweiten Teilbildes,
- Erfassen eines dritten Farbbildes eines iridokornealen Bereichs (101) des Auges (100), Auswählen eines dritten Teilbildes innerhalb des dritten Farbbildes, das dem Bereich von Interesse entspricht, und Berechnen einer dritten berechneten Farbe des dritten Teilbildes;
wobei zwischen dem Erfassen des zweiten Farbbildes und dem Erfassen des dritten Farbbildes eine Zeitspanne liegt;
umfassend die weiteren Prüfschritte:
- Berechnen eines Verhältnisses zwischen einem Abstand zwischen der dritten berechneten Farbe und der zweiten berechneten Farbe und einer Dauer des Zeitraums
- Bereitstellen dieses Verhältnisses als ein Prüfergebnis.

6. Verfahren nach Anspruch 5, umfassend vor den Erfassen des zweiten Farbbildes und dem Erfassen des dritten Farbbildes:
- Erfassen eines ersten Farbbildes eines iridokornealen Bereichs des Auges, Auswählen eines ersten Teilbildes innerhalb des ersten Farbbildes, das dem Bereich von Interesse entspricht, und Berechnen einer ersten berechneten Farbe des ersten Teilbildes.
Wobei zwischen dem Erfasssen des ersten Farbbildes und dem Erfassen des zweiten Farbbildes eine weitere Zeitspanne liegt.

7. Verfahren nach Anspruch 1,
wobei in Schritt D der Indikator basierend auf einer vorbestimmten Einstufungsskala bestimmt wird.

8. Verfahren nach Anspruch 7,
wobei in Schritt C eine Vielzahl von statistischen Parametern des Teilbildes berechnet wird,
wobei in Schritt D der Indikator basierend auf der Vielzahl von statistischen Parametern bestimmt wird, wobei Schritt D durch einen Klassifikator ausgeführt wird.

9. Verfahren nach Anspruch 7,
wobei in Schritt C eine Vielzahl von statistischen Parametern des Teilbildes berechnet wird,
wobei in Schritt D der Indikator basierend auf der Vielzahl von statistischen Parametern bestimmt wird, wobei Schritt D durch einen neuronalen Netzwerkklassifikator ausgeführt wird.

10. Verfahren nach Anspruch 9, ferner umfassend einen vorbereitenden Schritt des nur einmaligen oder wiederholten oder kontinuierlichen Schulens des neuronalen Netzwerkklassifikators.

11. Verfahren nach Anspruch 7 oder 8 oder 9 oder 10,
wobei in Schritt A eine Vielzahl von Farbbildern eines iridokornealen Bereichs (101) desselben Auges (100) unter verschiedenen Winkeln erfasst wird,
wobei in Schritt B eine entsprechende Vielzahl von Teilbildern aus der Vielzahl von Farbbildern ausgewählt wird,
wobei in Schritt C mindestens eine entsprechende Vielzahl von statistischen Parametern der Vielzahl von Teilbildern berechnet wird,
wobei in Schritt D eine entsprechende Vielzahl von Indikatoren basierend auf der mindestens einen Vielzahl von statistischen Parametern bestimmt wird.

12. Verfahren nach Anspruch 11,
wobei in Schritt E die Vielzahl von Indikatoren und/oder ein lineares oder kreisförmiges Diagramm, das die Vielzahl von Indikatoren zeigt, als Prüfergebnis bereitgestellt wird.

## Revendications

1. Méthode de création et de traitement d'une image couleur d'une région iridocornéenne (101) d'un œil (100) comprenant les étapes d'examen consistant à :
A) détecter au moins une image couleur d'une région iridocornéenne (101) d'un œil (100) par l'intermédiaire d'un dispositif détecteur (202) constitué d'un réseau bidimensionnel de pixels, l'au moins une image couleur se trouvant dans un espace couleur défini par trois dimensions de couleur, ainsi une couleur de chaque pixel dudit réseau bidimensionnel est associée à trois composantes de couleur, le dispositif détecteur (202) et un dispositif illuminateur de couleur (201) sont au préalable étalonnés en tant que paire,
B) sélectionner au moins une sous-image au sein de ladite image détectée correspondant à une région d'intérêt,
C) calculer au moins un paramètre statistique de ladite sous-image sélectionnée, le paramètre statistique étant choisi dans le groupe comprenant une valeur moyenne d'une composante de couleur, une valeur de variance d'une composante de couleur, une valeur minimale d'une composante de couleur et une valeur maximale d'une composante de couleur, une valeur d'asymétrie d'une composante de couleur, une valeur d'indice de kurtosis d'une composante de couleur, une distribution d'une composante de couleur, un gradient de distribution d'une composante de couleur,
D) déterminer un indicateur à partir dudit au moins un paramètre statistique,
E) fournir ledit indicateur en tant que résultat d'examen ;
dans laquelle l'étape A fournit une image couleur dans un espace couleur dépendant de l'appareil, à savoir RVB, et
dans laquelle avant l'étape C, il y a une étape F consistant à transformer la sous-image dans l'espace couleur dépendant du dispositif en une sous-image dans un espace couleur indépendant du dispositif et uniforme du point de vue de la perception, à savoir le CIE LAB, dans laquelle au moins les étapes B, C, D, E et F sont réalisées par un programme informatique.

2. Méthode selon la revendication 1,
dans laquelle l'étape C correspond au calcul d'au moins une couleur calculée de la sous-image,
dans laquelle l'étape D correspond au calcul d'au moins une distance entre ladite au moins une couleur calculée et au moins une couleur de comparaison,
dans laquelle l'étape E correspond à la fourniture de ladite distance au moins en tant que résultat d'examen.

3. Méthode selon la revendication 2, dans laquelle ladite au moins une couleur de comparaison est prédéterminée.

4. Méthode selon la revendication 3, dans laquelle ladite au moins une couleur de comparaison provient d'une campagne d'examen sur une pluralité de personnes.

5. Méthode selon l'une quelconque des revendications précédentes 2 à 4, comprenant les étapes d'examen consistant à :
- détecter une deuxième image couleur d'une région iridocornéenne (101) d'un œil (100), sélectionner une deuxième sous-image dans cette deuxième image couleur correspondant à une région d'intérêt, et calculer une deuxième couleur calculée de la deuxième sous-image,
- détecter une troisième image couleur d'une région iridocornéenne (101) de l'œil (100), sélectionner une troisième sous-image dans ladite troisième image couleur correspondant à ladite région d'intérêt, et calculer une troisième couleur calculée de la troisième sous-image ;
dans laquelle une période de temps s'écoule entre la détection de la deuxième image couleur et la détection de la troisième image couleur ;
comprenant les étapes d'examen supplémentaires consistant à :
- calculer un rapport entre une distance entre ladite troisième couleur calculée et ladite deuxième couleur calculée, et une durée de ladite période de temps
- fournir ce rapport en tant que résultat d'examen.

6. Méthode selon la revendication 5, comprenant, avant la détection de la deuxième image couleur et la détection de la troisième image couleur :
- la détection d'une première image couleur d'une région iridocornéenne de l'œil, la sélection d'une première sous-image dans ladite première image couleur correspondant à ladite région d'intérêt, et le calcul d'une première couleur calculée de la première sous-image.
Dans laquelle une autre période de temps s'écoule entre la détection de la première image couleur et la détection de la deuxième image couleur.

7. Méthode selon la revendication 1,
dans laquelle, à l'étape D, l'indicateur est déterminé sur la base d'une échelle de classement prédéterminée.

8. Méthode selon la revendication 7,
dans laquelle à l'étape C une pluralité de paramètres statistiques de la sous-image sont calculés,
dans laquelle à l'étape D l'indicateur est déterminé sur la base de la pluralité de paramètres statistiques, dans laquelle l'étape D est réalisée par l'intermédiaire d'un classificateur.

9. Méthode selon la revendication 7,
dans laquelle à l'étape C une pluralité de paramètres statistiques de la sous-image sont calculés,
dans laquelle à l'étape D l'indicateur est déterminé sur la base de plusieurs paramètres statistiques, dans laquelle l'étape D est réalisée par l'intermédiaire d'un classificateur de réseau neuronal.

10. Méthode selon la revendication 9, comprenant en outre une étape préliminaire consistant à former le classificateur de réseau neuronal une seule fois ou de manière répétée ou continue.

11. Méthode selon la revendication 7 ou 8 ou 9 ou 10,
dans laquelle à l'étape A une pluralité d'images couleur d'une région iridocornéenne (101) d'un même œil (100) sous différents angles sont détectées,
dans laquelle à l'étape B une pluralité correspondante de sous-images est sélectionnée dans ladite pluralité d'images couleur,
dans laquelle à l'étape C au moins une pluralité correspondante de paramètres statistiques de la pluralité de sous-images est calculée,
dans laquelle à l'étape D une pluralité correspondante d'indicateurs est déterminée sur la base d'au moins une pluralité de paramètres statistiques.

12. Méthode selon la revendication 11,
dans laquelle à l'étape E la pluralité d'indicateurs et/ou un graphique linéaire ou circulaire montrant la pluralité d'indicateurs sont fournis en tant que résultat d'examen.
